# FASCICULE DE BREVET EUROPEEN

(11) **EP 1 137 420 B1**
(45) Date de publication et mention de la délivrance du brevet: **04.04.2007**
(21) Numéro de dépôt: 99958233.1
(22) Date de dépôt: 02.12.1999
(51) Int. Cl.: A61K 31/70, A61K 31/535, A61K 31/52, A61K 31/195, A61P 15/10

(54) **UTILISATION D'UNE COMBINAISON D'UNE PURINE ET D'UN AGENT DONNEUR DE MONOXYDE D'AZOTE POUR PREVENIR OU A TRAITER LES DYSFONCTIONS SEXUELLES**
VERWENDUNG EINER KOMBINATION AUS EINEM PURIN UND EINEM NO-DONOR ZUR BEHANDLUNG UND PROPHYLAXE SEXUELLER FUNKTIONSSTÖRUNGEN
USE OF A COMBINATION OF A PURINE AND A NITROGEN MONOXIDE DONOR FOR PREVENTING OR TREATING SEXUAL DYSFUNCTION

(30) Priorité: 02.12.1998 FR 9815237
(43) Date de publication de la demande: 04.10.2001
(73) Titulaire: Adenobio N.V., 1118 BH Luchthaven Schiphol (NL)
(72) Inventeur: Gorny, Philippe, 75116 Paris (FR)
(74) Mandataire: Breese, Pierre
(86) Numéro de dépôt international: PCT/FR1999/002993
(87) Numéro de publication internationale: WO 2000/032202

(56) Documents cités:
- WO-A-00/00212
- WO-A-92/21346
- WO-A-94/03442
- WO-A-94/04120
- WO-A-97/39760
- WO-A-99/60985
- GB-A- 2 268 871
- US-A- 5 422 343
- US-A- 5 612 060
- PATENT ABSTRACTS OF JAPAN vol. 097, no. 009, 30 septembre 1997 (1997-09-30) & JP 09 124473 A (SNOW BRAND MILK PROD CO LTD), 13 mai 1997 (1997-05-13) & DATABASE WPI Week 9729 Derwent Publications Ltd., London, GB; AN 97-316471 & JP 09 124473 A (SNOW BRAND MILK PROD CO LTD), 13 mai 1997 (1997-05-13)
- PATENT ABSTRACTS OF JAPAN vol. 012, no. 455 (C-548), 29 novembre 1988 (1988-11-29) & JP 63 179827 A (FUSO YAKUHIN KOGYO KK), 23 juillet 1988 (1988-07-23) & DATABASE WPI Week 8835 Derwent Publications Ltd., London, GB; AN 88-246701 & JP 63 179827 A (FUSO YAKUHIN KOGYO KK), 23 juillet 1988 (1988-07-23)
- DATABASE WPI Week 9716, Derwent Publications Ltd., London, GB; AN 97-177462, XP002135773 & RU 2 064 792 C (MILITARY MED ACAD) 10 Août 1996

## Description

L'invention concerne l'obtention d'un médicament destiné à prévenir ou traiter les dysfonctions sexuelles de l'homme ou de la femme. L'invention concerne en particulier l'obtention d'un médicament capable de combattre les troubles de la réponse physiologique et/ou anatomique à la stimulation sexuelle chez les humains. Un tel médicament contient, en combinaison, une purine et un agent donneur de monoxyde d'azote.

On sait que, chez l'homme, le processus de l'érection est schématiquement celui indiqué ci-après. Le tissu érectile de la verge, appelé corps caverneux, est un tissu spongieux capable de se remplir de sang. Au repos, les artères de la verge sont sous la dépendance du tonus adrénergique qui les maintient spasmées, de sorte qu'aucun flux sanguin notable ne vient remplir le corps caverneux. En cas de stimulation appropriée, les nerfs érecteurs inhibent le tonus adrénergique, libèrent certains médiateurs favorisant la dilatation des artères de la verge, ce qui entraîne une accumulation de sang dans le corps caverneux. Ce dernier grossit, tandis que l'augmentation de sa pression interne fait qu'il devient rigide. En grossissant, il écrase les veines caverneuses contre l'enveloppe du corps érectile, empêchant l'évacuation du sang qu'il contient, ce qui assure le maintien de la rigidité. Après l'éjaculation, l'adrénaline est à nouveau libérée localement, l'apport en sang artériel se réduit aussitôt, la pression dans le corps caverneux diminue et le sang accumulé dans celui-ci peut s'évacuer par les veines qui ne sont plus comprimées, ce qui entraîne la perte de la rigidité, avec retour à l'état de repos.

Chez la femme, l'excitation sexuelle se traduit notamment par la vasodilatation des vaisseaux sanguins irriguant les organes génitaux. Cette vasodilatation entraîne en particulier un gonflement et une réponse érectile du clitoris, ainsi qu'une vasocongestion de la paroi vaginale avec exsudation de fluides vaginaux.

Il est connu qu'une proportion assez importante d'hommes (entre 10 et 50 % selon les populations étudiées et selon les tranches d'âge) souffre de dysfonctions érectiles permanentes ou temporaires. Ces troubles peuvent être d'origine organique, auquel cas ils nécessitent des traitements spécifiques adaptés à chaque cause. Mais on observe aussi une majorité de dysfonctions érectiles non organiques, souvent d'origine psychogène.

Chez les femmes également, la réponse physiologique à la stimulation sexuelle, et ses manifestations anatomiques, peuvent se trouver altérées temporairement, et parfois durablement, même sans cause organique décelable. Les troubles les plus fréquemment constatés incluent l'absence de désir sexuel même après stimulation, la difficulté d'obtenir un orgasme, la faible intensité du plaisir sexuel et la diminution de la lubrification vaginale naturelle ou même son absence. Ces troubles ont souvent pour conséquence le manque d'intérêt pour l'activité sexuelle. Ce sont ces troubles de la réponse physiologique et/ou anatomique à la stimulation sexuelle que l'on appelle, dans la présente demande, "dysfonctions sexuelles féminines". Selon certaines estimations, la fréquence des dysfonctions sexuelles temporaires ou chroniques, chez la femme, serait équivalente à celle des dysfonctions érectiles chez l'homme.

Il est donc souhaitable de disposer de traitements permettant de réduire la gravité et/ou la durée de ces troubles, ou d'en prévenir l'apparition, de restaurer la capacité d'accomplissement de rapports sexuels satisfaisants, chez les sujets, hommes ou femmes, qui présentent de tels troubles, ou qui en redoutent la survenue.

Pour les cas d'impuissance masculine, divers traitements ont été proposés. Dans les formes sévères, l'injection intracaverneuse de substances vasoactives est susceptible de fournir de bons résultats. En cas de dysfonctions érectiles modérées ou débutantes, les traitements par voie orale sont plus appropriés et sont généralement mieux acceptés. On a proposé à cet effet divers produits, souvent d'origine végétale. On a également proposé l'utilisation d'un vasodilatateur par voie orale (WO 96/33705 ; WO 96/16644), ou par voie transdermique, transmuqueuse, intra-nasale ou rectale (WO 95/05172).

Parmi les agents vasodilatateurs connus, on peut distinguer notamment les agents ayant des effets antagonistes sur les récepteurs α-adrénergiques (par exemple la phentolamine), qui inhibent le tonus adrénergique et favorisent ainsi la dilatation des artères, et les agents jouant le rôle de donneurs de monoxyde d'azote (NO), soit directement, soit au cours de leur métabolisme. On sait en effet que les cellules endothéliales, qui tapissent la face interne des vaisseaux sanguins, sont capables de sécréter une substance ayant pour effet de dilater les artères, cette substance étant le monoxyde d'azote. Il a été établi que le monoxyde d'azote stimule la synthèse de la guanosine monophosphate cyclique (ou GMPc) qui est l'agent effectif de la relaxation musculaire des artères. On sait aussi que le monoxyde d'azote est le principal neurotransmetteur physiologique mis en jeu par les neurones périphériques non adrénergiques et non cholinergiques innervant le corps caverneux et ses artères, et que sa libération au niveau de la synapse effectrice est un facteur important dans l'induction de l'érection ; voir notamment BURNETT *et al., Science* 257:401-403 (1992), et FAJFER *et al., New Engl. J. Med.* 326:90-94 (1992). La demande internationale WO 92/21346 préconise l'administration d'un donneur de monoxyde d'azote, la linsidomine, dans le traitement des dysfonctions érectiles.

Parmi les produits agissant sur la dilatation des artères par production de monoxyde d'azote, on peut citer par exemple l'arginine, le nitroprussiate de sodium, des nitrates organiques (trinitrate de glycérol, mononitrate ou dinitrate d'isosorbide), des nitrites organiques (nitrites d'amyle ou de butyle), des thionitrites tels que décrits dans le document WO 96/15645 (par exemple S-nitrosocystéine, S-nitrosoglutathion), la molsidomine, ainsi que, le cas échéant, les sels pharmaceutiquement acceptables de ces composés.

Il est connu que d'autres agents interviennent dans le phénomène physiologique de turgescence des corps érectiles. Parmi ces agents, on peut citer des prostaglandines, des peptides vasoactifs comme la bradykinine, le neuropeptide appelé peptide intestinal vaso-actif (ou VIP), le neuropeptide Y, etc. Par ailleurs, il a été montré chez le lapin que les purines sont capables d'induire une relaxation du corps caverneux ; voir WU *H-Y et al.* Int. J. Impotence Res. 5, 161-167 (1993). Il a également été montré que l'injection intraveineuse d'adénosine triphosphate induit l'érection chez le chien ; voir TAKAHASHI Y*. et al.* Int. J. Impotence Res. 4, 27-34 (1992). Les purines agiraient comme neurotransmetteurs non-adrénergiques non-cholinergiques.

On a maintenant découvert que la combinaison d'une activité de purine et d'une activité de donneur de monoxyde d'azote permet d'obtenir des résultats favorables dans la prévention et le traitement des troubles de la réponse physiologique et anatomique à la stimulation sexuelle chez les humains (homme ou femme), et donc de lutter contre lesdits troubles, grâce à un effet de synergie.

Dans la présente demande, on entend par "donneur de monoxyde d'azote" tout agent qui est capable de produire directement ou indirectement du monoxyde d'azote (NO) *in vivo,* ou de tout précurseur métabolique d'un tel agent, ainsi que tout agent susceptible de favoriser la production de monoxyde d'azote endogène. Par exemple, les inhibiteurs de phosphodiestérase ont pour effet indirect d'augmenter le taux de monoxyde d'azote. Les donneurs de monoxyde d'azote sont notamment les produits qui ont été mentionnés ci-dessus. Une activité de donneur de monoxyde d'azote est obtenue par la présence d'un tel agent.

Dans la présente demande, on entend par "purine" les bases puriques, notamment l'adénine, les nucléosides à base purique et notamment l'adénosine ainsi que les phosphates correspondants, notamment l'AMP, l'ADP et l'ATP, ainsi que leurs sels acceptables en pharmacie (par exemple chlorhydrate d'adénine ou d'adénosine, ou sels de sodium d'adénosine-phosphates). Plus généralement, on entend également par "purine" toute substance capable d'agir sur les récepteurs puriniques. De telles substances sont connues ou peuvent être recherchées selon des méthodes connues. Une activité de purine est une activité obtenue par la présence d'une purine telle qu'elle vient d'être définie.

Si un composé possède à la fois une activité de purine et une activité de donneur de monoxyde d'azote, il peut être utilisé comme ingrédient actif unique dans le médicament obtenu selon l'invention.

Pour étudier les effets des agents destinés à traiter les troubles de la réponse physiologique et anatomique à la stimulation sexuelle chez l'homme et chez la femme, on peut utiliser des méthodes connues telles que les tests qui sont décrits par BOOLELL M. *et al*., Intern. Journal of Impotence Research, 8, 47-52 (1996), ainsi que dans le document PCT WO 95/05172, ou dans les essais qui seront décrits ci-après.

On utilise le médicament obtenu conformément à l'invention de façon à administrer à la personne traitée des doses efficaces qui peuvent être déterminées par de simples expériences de routine, en utilisant par exemple les tests qui viennent d'être mentionnés. Il convient d'ailleurs de remarquer que plusieurs purines actives et de nombreux donneurs de monoxyde d'azote sont connus, de même que leurs doses actives. Il est en outre facile de déterminer les doses efficaces à l'aide de tels tests. En comparant les effets de l'association avec les effets de chacun de ses ingrédients actifs, on constate que l'association permet en général de réduire les doses de l'un au moins des ingrédients actifs. On peut ainsi sélectionner les associations présentant un effet de synergie.

L'invention a donc pour objet l'utilisation en combinaison d'une purine et d'un agent donneur de monoxyde d'azote conformes à la revendication 1, comme ingrédients actifs dans la préparation d'un médicament destiné à prévenir ou traiter les troubles de la réponse physiologique et/ou anatomique à la stimulation sexuelle, et en particulier à prévenir ou traiter les dysfonctions érectiles non organiques. Ce médicament est administré à des sujets qui en ont besoin, c'est-à-dire les personnes ayant éprouvé ou redoutant de tels troubles.

Les ingrédients actifs d'un médicament obtenu conformément à l'invention peuvent être présentés de façon séparée, chacun sous une forme pharmaceutique appropriée, et réunis dans un même emballage.

Mais pour faciliter l'administration simultanée des ingrédients actifs, on préfère généralement préparer le médicament sous une seule forme pharmaceutique contenant les deux ingrédients actifs, ou l'ingrédient actif unique dans le cas où celui-ci possède les deux types d'activités (activité purinique et activité de donneur de monoxyde d'azote), ainsi éventuellement qu'un excipient pharmaceutique approprié.

Le médicament obtenu conformément à l'invention peut être administré par voie orale, sublinguale, nasale, pulmonaire, vaginale, rectale ou transdermique, ou encore par injection intracaverneuse.

A cet effet, il peut être présenté sous toute forme permettant l'administration par voie orale (en particulier sous la forme de gélules, de solutions ou émulsions buvables, de poudres, de gels, de granulés, de tablettes ou de comprimés), par voie nasale (par exemple des solutions à administrer sous forme de gouttes ou de pulvérisations), par voie pulmonaire (solutions en flacon pressurisé pour aérosols), par voie rectale (suppositoires), par voie cutanée (par exemple onguents ou dispositifs transdermiques, encore appelés timbres ou patches), ou par voie transmuqueuse comme par exemple par voie sublinguale (solutions en flacon pressurisé, ou comprimés à délitement buccal) ou par voie vaginale (notamment crèmes ou ovules gynécologiques), ou encore par voie intracaverneuse (suspensions ou solutions injectables).

Ces formes pharmaceutiques sont préparées de façon usuelle et peuvent contenir des excipients et véhicules classiques appropriés.

Les médicaments obtenus conformément à l'invention sont notamment ceux qui sont exempts d'adénosine, ainsi que ceux qui sont exempts de linsidomine.

Parmi les associations utilisées conformément à l'invention, on citera notamment celle de l'adénosine monophosphate (AMP), ou de l'adénosine triphosphate (ATP), avec l'arginine.

La L-arginine est un précurseur du monoxyde d'azote endogène, et son administration se traduit notamment par un effet sur la relaxation musculaire des artères et du corps caverneux, cette relaxation étant nécessaire à l'obtention de l'érection. L'administration de 2800 mg par jour de L-arginine aurait un effet favorable sur les dysfonctions érectiles dans 40 % des cas environ ; voir A.W. ZORGNIOTTI et E.F. LIZZA, Int. J. Impotence Res., 6, 33-36 (1994).

L'arginine peut être utilisée sous forme non salifiée, ou sous forme salifiée (notamment sous forme de chlorhydrate, de glutamate, d'aspartate ou de citrate).

Le médicament de l'invention permet d'obtenir des résultats favorables chez des hommes souffrant de dysfonctions érectiles passagères, et aussi chez des sujets atteints de dysfonctions érectiles chroniques. Chez les femmes, des améliorations sont constatées notamment pour l'un au moins des troubles suivants : perte ou diminution du désir sexuel, absence d'orgasme ou difficulté à obtenir un orgasme, sécheresse vaginale, diminution de l'intensité du plaisir sexuel, etc.

Le médicament obtenu conformément à l'invention peut être utilisé soit sur de longues périodes dans les cas de dysfonctions érectiles chroniques (par exemple cures de plusieurs semaines, plusieurs fois par an), soit en cures épisodiques dans le traitement des dysfonctions érectiles temporaires et/ou récentes, soit encore de façon ponctuelle.

Un tel médicament peut être préparé par exemple sous une forme pharmaceutique permettant l'administration de 30 à 150 mg d'AMP en une ou deux prises, et permettant en outre l'administration d'une dose suffisante d'arginine, par exemple une dose de 1 à 8 g par jour, le plus souvent de 1,5 à 3 g, en une ou deux prises, ladite dose étant calculée en poids d'arginine sous forme de base libre.

Par exemple, on peut administrer une dose de 50 à 100 mg d'AMP et de 1 à 2 g par jour de L-arginine, chez l'adulte, pour un traitement devant durer de 2 à 4 semaines. Dans le cas d'une utilisation ponctuelle, on peut administrer, par exemple par voie orale ou sublinguale, de 60 à 120 mg d'AMP, et de 1,5 à 4 g de L-arginine en une seule prise, environ 30 minutes à 2 heures avant un rapport sexuel envisagé.

On peut remplacer l'AMP notamment par des quantités équivalentes d'ATP.

L'invention concerne également une méthode de prévention ou de traitement des dysfonctions sexuelles masculines ou féminines, dans laquelle on administre un médicament tel que défini ci-dessus.

Des essais ont été effectués sur des femmes volontaires âgées de 30 à 55 ans, souffrant de l'un au moins des troubles suivants : perte du désir sexuel, impossibilité d'obtenir un orgasme, diminution de l'intensité du plaisir sexuel, ou sécheresse vaginale. On a remis aux personnes testées des sachets contenant 3 g de glutamate d'arginine et 60 mg d'AMP sous forme de poudre à mettre en suspension dans l'eau, en leur demandant d'ingérer le contenu d'un sachet de poudre une fois par jour pendant deux semaines.

Les personnes testées ont noté par auto-évaluation les effets observés pendant le traitement, en ce qui concerne le désir sexuel, l'obtention d'un orgasme, l'intensité du plaisir sexuel et la lubrification vaginale. Une majorité de personnes testées a constaté une amélioration sur au moins deux des critères retenus dans cette étude.

Par ailleurs, des essais analogues ont été effectués sur des hommes âgés de 38 à 70 ans, souffrant d'une dysfonction sexuelle débutante. On leur a demandé d'absorber le contenu d'un sachet de poudre par jour pendant 10 jours, et, en outre, d'ingérer ponctuellement le contenu d'un sachet supplémentaire, 30 minutes à deux heures avant une activité sexuelle envisagée. Environ 60 % des personnes testées ont constaté pendant le traitement une amélioration de la qualité ou de la fréquence de survenue de l'érection.

L'exemple suivant illustre l'invention.

### EXEMPLE : Sachets de poudre pour suspensions buvables

On prépare des sachets de poudre contenant :
- AMP : 60 mg
- Glutamate d'arginine : 3 g
- Excipient aromatisé : 0,5 g

On peut remplacer l'AMP par une quantité équivalente d'ATP.

On préconise d'ingérer chaque jour le contenu d'un sachet, après mise en suspension dans l'eau. On peut en outre ingérer le contenu d'un sachet supplémentaire 30 minutes à 2 heures avant une activité sexuelle envisagée.

## Revendications

1. Utilisation, en combinaison, d'une purine choisie parmi l'adénine, l'AMP, l'ADP et l'ATP, et d'un agent donneur de monoxyde d'azote choisi parmi l'arginine, le nitroprussiate de sodium, des nitrates ou ou nitrites organiques, des thionitrites et la molsidomine, ainsi que le cas échéant les sels pharmaceutiquement acceptables de ces composés, comme ingrédients actifs dans la préparation d'un médicament destiné à prévenir ou traiter les troubles de la réponse physiologique et/ou anatomique à la stimulation sexuelle chez les humains.

2. Utilisation selon la revendication 1, **caractérisée en ce que** ledit médicament contient lesdits ingrédients actifs, de façon séparée, dans un même emballage.

3. Utilisation selon la revendication 1, **caractérisée en ce que** ledit médicament se présente sous une forme pharmaceutique unique.

4. Utilisation selon l'une quelconque des revendications précédentes, **caractérisée en ce que** ledit médicament se présente sous la forme de gélules, de solutions ou émulsions buvables, de granulés, de gels, de crèmes, de poudres, de tablettes, de comprimés, d'onguents, de dispositifs transdermiques, d'ovules gynécologiques, de suppositoires, ou de solutions, éventuellement en flacons pressurisés, pour administration par voie nasale ou pulmonaire, ou de solutions ou suspensions injectables par voir intracaverneuse.

5. Utilisation selon l'une quelconque des revendications précédentes, dans laquelle ladite purine est l'AMP ou l'ATP.

6. Utilisation selon l'une quelconque des revendications précédentes, dans laquelle on prépare ledit médicament sous une forme pharmaceutique permettant l'administration d'une dose de 30 à 150 mg de purine, en une ou deux prises.

7. Utilisation selon l'une quelconque des revendications précédentes, dans laquelle l'agent donneur de monoxyde d'azote est l'arginine.

8. Utilisation selon la revendication précédente, dans laquelle on prépare ledit médicament sous une forme pharmaceutique permettant l'administration d'une dose de 1 à 8 g d'arginine, et en particulier de 1,5 à 3 g, en une ou deux prises, ladite dose étant calculée en poids d'arginine sous forme de base libre.

9. Utilisation selon l'une quelconque des revendications précédentes, dans laquelle ladite purine est l'AMP ou l'ATP et ledit agent donneur de monoxyde d'azote est l'arginine.

## Claims

1. Use of a purine chosen from among adenine, AMP, ADP and ATP in combination with a nitrogen monoxide donor agent chosen from among arginine, sodium nitroprussiate, organic nitrates or nitrites, thiontrites and molsidomine, and possibly pharmaceutically acceptable salts of these compounds as active ingredients in the preparation of a medicine designed to prevent or to treat physiological and/or anatomic response disorders to sexual stimulation in humans.

2. Use set forth in claim 1, **characterised in that** said medicine contains said active ingredients, separately, in the same packaging.

3. Use set forth in claim 1, **characterised in that** the said medicine is in a single pharmaceutical form.

4. Use set forth in any one of the above claims, **characterised in** said medicine is in the form of gelatine capsules, drinkable solutions or emulsions, granules, gels, creams, powders, tablets, pills, ointments, transdermic devices, vaginal suppositories, anal suppositories or solutions (possibly in pressurised flasks) for administration by nasal or lung spray, or intracavernously injected solutions or suspensions.

5. Use set forth in any one of the above claims, in which said purine is AMP or ATP.

6. Use set forth in any one of the above claims, in which said medicine is prepared in a pharmaceutical form enabling the administration of 20 to 150 mg of purine, in one or two doses.

7. Use set forth in any one of the above claims, in which the nitrogen monoxide donor agent is arginine.

8. Use set forth in any one of the above claims, in which said medicine is prepared in a pharmaceutical form enabling the administration of 1 to 8 g of arginine, and particularly between 1.5 and 3 g in one or two intakes, said dose being calculated by weight of arginine in the form of a free base.

9. Use set forth in any one of the above claims, in which said purine is AMP or ATP, and said nitrogen monoxide donor agent is arginine.

## Patentansprüche

1. Verwendung, in Kombination eines Purins, ausgewählt aus Adenin, AMP, ADP und ATP, und eines Stickstoffmonoxid-Donors, ausgewählt aus Arginin, Natriumnitroprussiat, Nitraten oder organischen Nitriten, Thionitriten und Molsidomin, sowie gegebenenfalls den pharmazeutisch akzeptablen Salzen dieser Verbindungen, als aktive Inhaltsstoffe in der Zubereitung eines Medikaments, das dazu bestimmt ist, Störungen der physiologischen und/oder anatomischen Antwort auf die sexuelle Stimulierung beim Menschen vorzubeugen oder zu behandeln.

2. Verwendung nach Anspruch 1, **dadurch gekennzeichnet, dass** das besagte Medikament die besagten aktiven Inhaltsstoffe separat in derselben Verpackung enthält.

3. Verwendung nach Anspruch 1, **dadurch gekennzeichnet, dass** sich das besagte Medikament in einer pharmazeutischen Einheitsform darstellt.

4. Verwendung nach einem der vorhergehenden Ansprüche, **dadurch gekennzeichnet, dass** sich das besagte Medikament in Form von Gelatinekapseln, trinkbaren Lösungen oder Emulsionen, Granulaten, Gelen, Cremes, Pulvern, Tabletten, Pastillen, Salben, transdermalen Vorrichtungen, gynäkologischen Kugeln, Zäpfchen oder Lösungen, eventuell in Flakons unter Druck zur Verabreichung über die Nase oder die Lungen, oder von Lösungen oder Suspensionen, die auf intrakavernösem Weg injizierbar sind, darstellt.

5. Verwendung nach einem der vorhergehenden Ansprüche, in der das besagte Purin AMP oder ATP ist.

6. Verwendung nach einem der vorhergehenden Ansprüche, in welcher das besagte Medikament in einer pharmazeutischen Form zubereitet wird, welche die Verabreichung einer Dosis von 30 bis 150 mg Purin in einer oder zwei Einnahmen erlaubt.

7. Verwendung nach einem der vorhergehenden Ansprüche, in welcher der Stickstoffmonoxid-Donor Arginin ist.

8. Verwendung nach dem vorhergehenden Anspruch, in welcher das besagte Medikament in einer pharmazeutischen Form zubereitet wird, welche die Verabreichung einer Dosis von 1 bis 8 g Arginin und insbesondere von 1,5 bis 3 g in einer oder zwei Einnahmen erlaubt, wobei die besagte Dosis in Gewicht des Arginins in frei basierter Form berechnet wird.

9. Verwendung nach einem der vorhergehenden Ansprüche, in welcher das besagte Purin AMP oder ATP und der besagte Sickstoffmonoxid-Donor Arginin ist.
